# FASCICULE DE BREVET EUROPEEN

(11) **EP 1 842 485 B1**
(45) Date de publication et mention de la délivrance du brevet: **10.06.2009**
(21) Numéro de dépôt: 07290408.9
(22) Date de dépôt: 04.04.2007
(51) Int. Cl.: A61B 5/0408, A61B 5/0416

(54) **Ceinture thoracique à usage unique pour le recueil de signaux physiologiques d'activité cardiaque**
Brustgürtel zum ausschließlichen Einsatz für die Aufzeichnung physiologischer Signale der Herzaktivität
Disposable thoracic belt for collecting physiological signs of heart activity

(30) Priorité: 06.04.2006 FR 0603047
(43) Date de publication de la demande: 10.10.2007
(73) Titulaire: ELA MEDICAL, 92541 Montrouge (FR)
(72) Inventeur: Faisandier, Yves, 75116 Paris (FR)
(74) Mandataire: Dupuis-Latour, Dominique

(56) Documents cités:
- EP-A1- 0 548 435
- US-A- 4 026 278
- US-A- 4 706 680
- US-A- 5 622 168
- US-A1- 2002 103 513
- US-A1- 2002 138 125

## Description

L'invention concerne les appareils pour l'enregistrement de signaux physiologiques d'activité cardiaque (ECG) recueillis par des électrodes externes appliquées à un patient.

L'invention vise plus particulièrement les appareils portatifs de ce type destinés à l'enregistrement ambulatoire, en continu et sur une longue période des événements cardiaques. Cette technique utilise traditionnellement un boîtier enregistreur comportant une mémoire, sur lequel se connectent des câbles se fixant sur les électrodes.

L'une des difficultés de mise en oeuvre de cette technique réside dans le positionnement correct des électrodes et leur maintien en place pendante toute la durée de l'enregistrement, qui peut atteindre plusieurs jours ou plusieurs semaines, voire plusieurs mois.

Il a été proposé, notamment par le US-A-2005/0049515 d'incorporer les électrodes de recueil à une ceinture thoracique pouvant également supporter l'enregistreur, l'ensemble étant ainsi maintenu en place avec le minimum d'inconfort pour le patient.

Le US-A-5 622 168 décrit également une ceinture de ce type, correspondant au préambule de la revendication 1, où la bande support comporte une pluralité d'électrodes de recueil en forme de pastilles de gel solide, disposées en des emplacement prédéterminés côté interne de la bande support, ainsi qu'une pluralité correspondante d'organes de connexion électrique en forme de boutons-pression métalliques comprenant chacun un élément formant boule disposé côté externe de la bande support, serti sur un élément formant base disposé côté interne de la bande support, pour la connexion de chacune des électrodes à une entrée de signal respective d'un enregistreur d'événements cardiaques.

La structure proposée par ce document rend cependant la réalisation de cette ceinture thoracique relativement onéreuse.

Le US-A-4 706 680 décrit une électrode médicale comprenant une pastille de gel hydrophile solide, avec une languette attenante portant un bouton-pression permettant la connexion à un enregistreur. Le gel présente en outre un caractère fortement adhésif, pour permettre la fixation directe de l'électrode sur la peau du patient et son maintien sous risque de décollement accidentel.

L'un des buts de la présente invention est de proposer une nouvelle ceinture thoracique de ce type, mais dont la conception permette de la fabriquer à très bas coût, autorisant une utilisation unique : la ceinture sera alors considérée comme un simple consommable, jeté après usage ou échangé par le patient lui-même après quelques jours de port, dans le cas d'un enregistrement de très longue durée.

Un autre but de l'invention est de proposer une telle ceinture qui, en outre et surtout, ne développe pas de phénomènes allergiques contrairement à la majorité des électrodes adhésives, qui ne sont jamais réellement inertes, même celles prévues pour les longues durées. Cet aspect est d'autant plus important que la ceinture est destinée à être portée pendant une très longue durée, de plusieurs semaines et même plus.

La ceinture de l'invention est du type général précité connu d'après le US-A-5 622 168 précité, et comprend les caractéristiques énoncées dans la revendication 1.

Cette configuration où la languette de contact de l'électrode et la bande support sont simultanément prises en sandwich entre les deux éléments du bouton-poussoir évite en particulier de coller l'électrode sur le dos du bouton-pression, comme dans le cas du US-A-5 622 168.

Les sous-revendications énoncent diverses caractéristiques subsidiaires avantageuses.

On va maintenant décrire un exemple de mise en oeuvre du dispositif de l'invention, en référence aux dessins annexés où les mêmes références numériques désignent d'une figure à l'autre des éléments identiques.
La figure 1 est une vue schématique montrant la manière dont la ceinture est portée par le patient.
Les figures 2 et 3 sont des vues, respectivement en plan et en élévation, de la ceinture de l'invention.
Les figures 4 à 6 sont des vues en plan partielles illustrant diverses étapes successives de la réalisation de la ceinture de l'invention.
Les figures 7 à 10 sont des vues en coupe agrandies, partielles, montrant des étapes successives de la réalisation de la ceinture de l'invention.
La figure 11 illustre, isolément, la pièce de maintien et de protection, avec les régions revêtues de colle et la fenêtre formée à l'endroit de l'électrode.

On va maintenant décrire en détail un exemple de réalisation de la ceinture thoracique selon l'invention, ainsi que son procédé de fabrication.

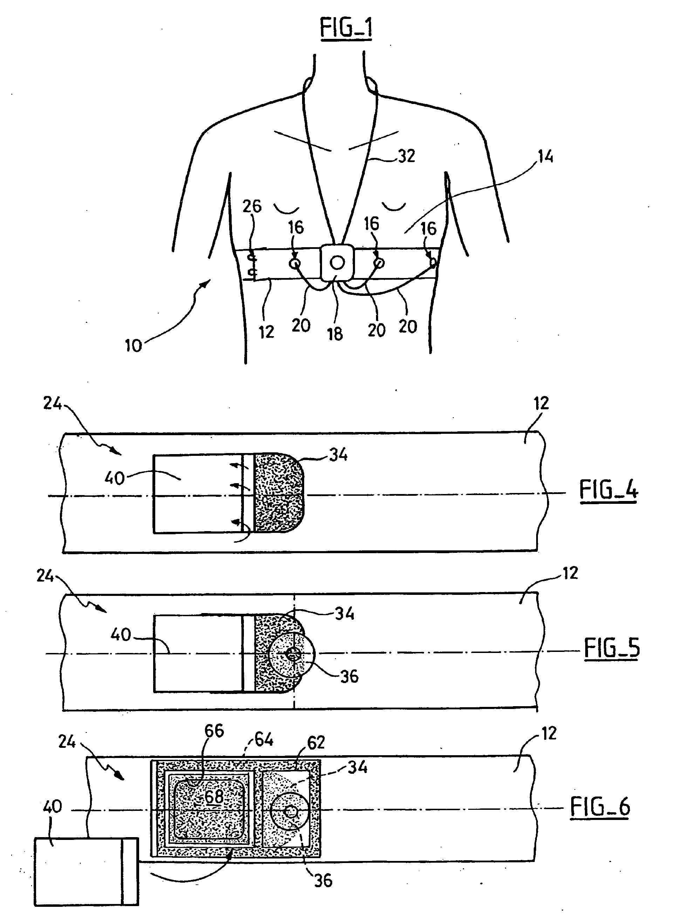

Sur la figure 1, on a représenté schématiquement en 10 une telle ceinture thoracique, formée d'une bande support souple 12 mise en place sur le thorax 14 du patient. Cette ceinture porte une pluralité d'électrodes 16 reliées à un enregistreur 18 par l'intermédiaire de câbles 20. L'appareil utilisé est par exemple un enregistreur du type *SpiderFlash* à trois électrodes, de ELA Medical. Cet appareil est du type "enregistreur d'événements", c'est-à-dire destiné à recueillir des signaux sur des très longues périodes (typiquement de plusieurs jours à plusieurs mois). Il peut notamment être conçu pour n'enregistrer les signaux que :
- lors d'un appui sur le bouton de déclenchement placé sur le boîtier, par le patient lui-même lorsqu'il ressent un symptôme ; si l'appareil dispose d'une mémoire en boucle, le signal enregistré peut contenir les informations qui précèdent l'appui de quelques secondes à quelques minutes,
- à heures programmées,
- ou de façon sélective en fonction des résultats d"ne analyse permanente du signal avec reconnaissance des troubles du rythme ou autre, si l'appareil dispose d'un module approprié.

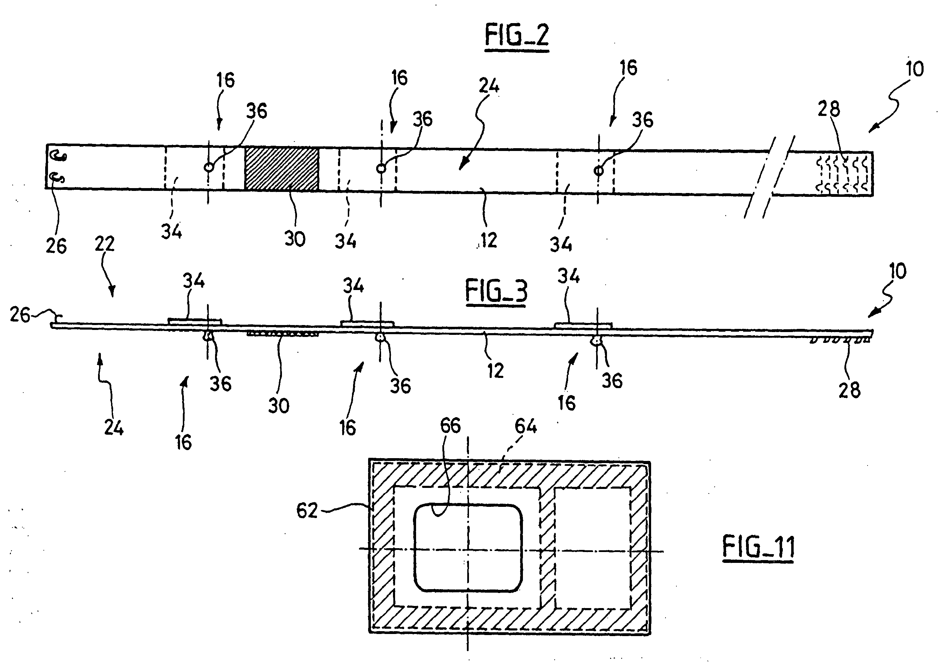

La ceinture proprement dite est représentée plus en détail sur les figures 2 et 3, sous forme déroulée. On désignera par "côte interne" 22 le côté destiné à venir en contact avec la peau du patient, et "côté externe" 24 le côté opposé. La ceinture est dotée d'un système d'accrochage, par exemple des crochets 28 situés côté interne à l'une des extrémités, coopérant avec des rangées de boucles 28 situées côté externe à l'extrémité opposée, pour relier entre elles les deux extrémités, avec possibilité de réglage (par choix de la rangée de boucles 28) pour adapter exactement la longueur de la ceinture au périmètre du thorax du patient. Pour pouvoir s'adapter à toutes les morphologies, il est cependant nécessaire de recourir à une gamme de ceintures de longueurs différentes, la longueur d'une ceinture de la gamme pouvant ainsi être réglée par exemple à 1 cm près avec un débattement de 9 cm.

La bande souple 12 est par exemple une bande de coton élastique de 30 mm de large comportant côté externe, outre les moyens de fixation précités, une pièce de matière agrippante 30, ou un sachet, pour permettre la fixation amovible de l'enregistreur. L'enregistreur peut ainsi être supporté par la ceinture, éventuellement avec l'aide d'un collier de soutien 32 (figure 1). On peut aussi laisser l'enregistreur dans une pochette fixée autour du cou, indépendante de la ceinture. Le patient perd alors un peu de confort lorsqu'il enlève et remet la ceinture, mais cette solution évite de créer sur le signal des artefacts liés aux contraintes mécaniques lorsque le patient appuie sur le bouton de déclenchement d'enregistrement de l'appareil.

Pour que le patient puisse accéder au système d'accrochage sans faire tourner la ceinture autour de lui (la ceinture étant bloquée par l'adhésion des électrodes), le système d'accrochage est placé en position légèrement antérieure, par exemple au niveau avant droit.

Les électrodes 16 sont constituées chacune d'une électrode de recueil proprement dite 34, placée côté interne pour venir en contact avec la peau du patient, combinée à un bouton-pression 36 électriquement relié à l'électrode 34 et permettant de clipser côté externe le fil de liaison 20 pour relier cette électrode à l'entrée de signal correspondante de l'enregistreur 18.

La position des électrodes sur la bande support 12 est déterminée de manière que, lorsque la ceinture est correctement positionnée, avec l'enregistreur centré à l'avant du patient, les électrodes se situent en face du coeur aux points de recueil du signal. Avec trois électrodes, on peut ainsi recueillir la tension entre l'électrode centrale et l'électrode droite correspondant à une dérivation de type I (droite-gauche), et la tension entre l'électrode centrale et l'électrode gauche correspondant à une dérivation de type V1-V5.

Compte tenu de l'usage recherché, l'électrode 34 doit être une électrode de type repositionnable, de façon à pouvoir enlever et remettre la ceinture. Plutôt que des électrodes sèches en métal ou autre matériau conducteur présentant un faible potentiel de contact avec la peau, les électrodes sont avantageusement des électrodes comprenant un gel légèrement adhésif, permettant d'assurer un excellent contact et engendrant un minimum d'artefacts sur les signaux recueillis.

L'utilisation de la ceinture est extrêmement simple, et peut être effectuée par le patient lui-même.

L'ensemble est conditionné par exemple dans une pochette métallisée étanche permettant de garder un niveau d'humidité évitant la dessiccation du gel des électrodes. Le patient retire la ceinture de son conditionnement, fixe l'enregistreur sur la bande agrippante prévue à cet effet, et raccorde les trois fils aux trois électrodes correspondantes (il peut être prévu par exemple un système de détrompage avec des couleurs différentes pour éviter toute confusion), puis l'enregistreur est mis en route.

La ceinture est alors mise en place sur le thorax et centrée, en positionnant l'enregistreur au niveau du sternum : les électrodes viennent alors se placer spontanément aux emplacements prédéfinis.

Le patient peut retirer la ceinture à tout moment lorsqu'il le souhaite, par exemple pour prendre une douche ou même la nuit dans le cas où l'on ne cherche à enregistrer que des symptômes diurnes.

On va maintenant décrire de façon plus détaillée un exemple d'une telle ceinture et son procédé de fabrication, en référence aux figures 4 à 11.

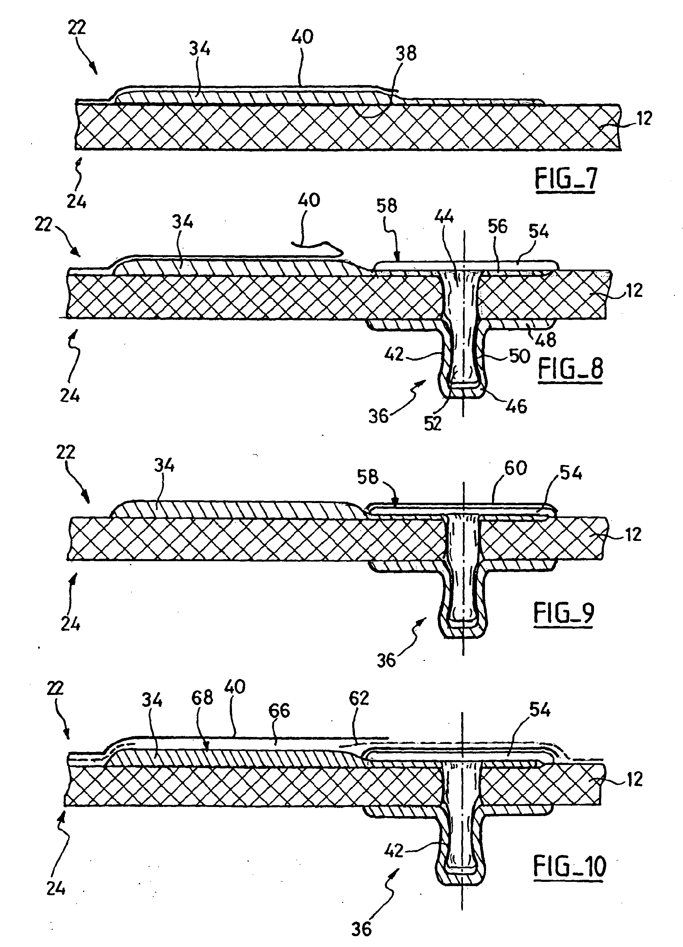

La première étape, illustrée figures 4 et 7, consiste à coller les électrodes 34 sur la bande 12 aux emplacements prescrits.

Les électrodes utilisées peuvent être par exemple du type *Blue Sensor BS* 3400 commercialisées par la société Ambu A/S (Medicotest A/S). Ces électrodes se présentent sous forme d'une pastille de gel solide à base argent/chlorure d'argent, non allergique, de dimensions 33 x 21 mm, avec une base formée d'un film métallique 38 recouvert par le gel adhésif et prolongé latéralement en une languette aplatie de branchement ou de soudage à froid d'un fil de liaison. Ces électrodes sont conditionnées par plaques de dix sur une feuille de papier siliconé dont elles peuvent être directement détachées. Sur la face supérieure, le gel adhésif est protégé par un film 40 en matière plastique.

Ces électrodes sont détachées de leur support et placées sur la bande en coton de la ceinture aux endroits prescrits. Les électrodes sont collées sur le coton de la ceinture au moyen d'une colle polymérisable qui devient neutre après durcissement. Si la colle n'est pas biocompatible, il est nécessaire de s'assurer qu'elle ne peut entrer en contact avec la peau du patient. Dans le cas contraire, il est nécessaire d'utiliser une colle de type "medical grade". Enfin, pour préparer l'étape suivante, le film de protection 40 du gel est partiellement relevé.

L'étape suivante, illustrée figures 5 et 8, consiste à monter, en contact avec chacune des électrodes 34, le bouton-pression 36 qui lui est associé.

De manière classique, un tel bouton-pression est constitué d'un élément formant boule 42 et d'un élément formant base 44, assemblés ensemble par sertissage. L'élément formant boule 42 comprend une partie renflée 46 sur laquelle pourra se clipser le fil de liaison à l'enregistreur, et une partie aplatie en forme de disque 48, avec entre les deux une tige intermédiaire 50, plus étroite que la partie arrondie 46.

L'élément formant base 44 comprend une tige 52 sertie à l'intérieur de l'élément formant boule 42, et une partie aplatie en forme de disque 54 venant pincer la région de contact 56 de l'électrode 34 contre la bande 12.

On choisit pour le matériau du bouton-pression, notamment pour le matériau de l'élément formant base, un matériau biocompatible tel que l'acier inoxydable ou un alliage dépourvu de nickel, de préférence relativement mou pour garantir un bon contact avec l'électrode.

Une fois le bouton-pression 36 positionné sur le bord de l'électrode avec la boule placée côté extérieur, le sertissage s'effectue avec les outils habituels en évitant toute déformation de la boule, la force de sertissage étant ajustée pour éviter d'abîmer le film métallique 38 à la base de l'électrode.

L'étape suivante, illustrée figure 8, consiste à isoler la face apparente 58 de la région aplatie 54 de l'élément formant base, en y collant une pastille isolante 60 ou en y appliquant une couche de vernis biocompatible et isolant. Il est en effet nécessaire d'éviter les réactions chimiques entre la peau et le bouton-pression, qui pourraient créer des artefacts de niveau élevé sur le signal recueilli en provenance de l'électrode 34.

L'étape suivante, illustrée figures 6 et 10, consiste à poser un film de maintien et de protection de l'électrode. Ce film se présente par exemple sous forme d'une pièce rectangulaire 62 telle que celle illustrée figure 11, de largeur légèrement supérieure à celle de l'électrode, et de longueur permettant de couvrir la totalité de la surface de l'électrode et du bouton-pression. Il est possible d'utiliser pour cette pièce 62 par exemple un matériau non tissé portant une enduction adhésive 64 limitée à la périphérie de la région de l'électrode et du bouton-pression. Pour assurer un maintien satisfaisant, il es souhaitable que l'enduction adhésive 64 couvre aussi une étroite bande périphérique du gel de l'électrode. Une fenêtre 66 est prévue pour que la surface active 68 de l'électrode puisse effectivement venir en contact avec la peau du patient.

En variante, il est possible d'utiliser pour cette pièce 62 un matériau très lâche, par exemple un matériau fibreux de type gaze, permettant d'ancrer le gel sans pour autant nuire au contact entre ce gel et la peau du patient.

Enfin, le film de protection 40 est remis en place, de manière à éviter toute dessiccation du gel.

## Revendications

1. Une ceinture thoracique (10) pour le recueil de signaux physiologiques d'activité cardiaque, comprenant une bande support souple (12) apte à être fermée en boucle sur le thorax (14) d'un patient, définissant alors un côté interne (22) en contact avec le corps du patient et un côté externe (24) opposé, cette bande support comportant :
- une pluralité d'électrodes de recueil (16) en forme de pastilles de gel solide (34), disposées en des emplacement prédéterminés côté interne de la bande support, ainsi que
- une pluralité correspondante d'organes de connexion électrique (36) en forme de boutons-pression métalliques comprenant chacun, pour la ' connexion de chacune des électrodes à une entrée de signal respective d'un enregistreur d'événements cardiaques (18) :
• un élément formant boule (42), disposé côté externe de la bande support, comportant une partie renflée (46) apte à recevoir par clip-sage un fil de liaison audit enregistreur, et
• un élément formant base (44), disposé côté interne de la bande support, comportant une tige (52) sertie à l'intérieur de l'élément formant boule,
ceinture **caractérisée en ce que** :
- les électrodes (34) sont prolongées latéralement par une languette aplatie apte à l'établissement d'une connexion électrique, et comprenant une région de contact (56) interposée entre la face interne de la bande (12) et la face en vis-à-vis de l'élément formant base (44) du bouton-pression respectif, de manière à établir une liaison électrique entre le bouton-pression et le gel de l'électrode, et
- l'élément formant boule (42) et l'élément formant base (44) des boutons-pression enserrent à la fois ladite languette aplatie et la bande support (12), en pinçant la région de contact (56) de l'électrode (34) contre la bande support (12), et
- il est prévu côté interne une couche de maintien et de protection (62) couvrant la périphérie de l'électrode et comportant une fenêtre centrale (66) laissant apparente la partie libre formant surface active (68), tournée vers le corps du patient, de l'électrode (34).

2. La ceinture thoracique de la revendication 1, où ladite couche de maintien et de protection (62) couvre en outre la partie libre, tournée vers le corps du patient, de l'élément formant base (44) du bouton-pression.

3. La ceinture thoracique de la revendication 1, où ladite couche de maintien et de protection est en un matériau à texture lâche de type gaze ne formant pas barrière entre le gel de l'électrode et le corps du patient lorsque la ceinture est placée au contact du thorax du patient.

4. La ceinture thoracique de la revendication 1, où la couche de maintien et de protection (62) est, au moins dans la région (64) s'étendant en périphérie de l'électrode, enduite d'une matière adhésive sur sa surface tournée vers l'électrode et la bande support.

5. La ceinture thoracique de la revendication 1, comprenant en outre côté interne une pellicule amovible isolante (40) couvrant au moins la partie libre (68), tournée vers le corps du patient, de l'électrode.

6. La ceinture thoracique de la revendication 1, comprenant en outre côté externe, en position antérieure, un moyen (30) de fixation d'un boîtier d'enregistreur d'activité cardiaque.

7. La ceinture thoracique de la revendication 1, comprenant aux extrémités de la bande support un moyen (26, 28) de fermeture et d'ajustement de longueur de la ceinture.

8. La ceinture thoracique de la revendication 1, où ledit gel solide est un gel adhésif.

9. La ceinture thoracique de la revendication 1, comprenant côté interne une couche d'un revêtement isolant (60) couvrant la partie libre (58), tournée vers le corps du patient, de l'élément formant base du bouton-pression.

## Claims

1. A thoracic belt (10) for collecting physiological cardiac activity signals, comprising a flexible support band (12) able to be closed with a buckle on a patient's thorax (14), then defining an internal side (22) in contact with patient's body and an opposed external side (24), wherein said support band comprises :
- a plurality of collecting electrodes (16) in form og solid gel pastilles (34), disposed on predetermined places on the internal side of the support band, and
- a corresponding plurality of electrical connection members (36) in form of metallic pushbuttons, each of them comprising for connecting each of the electrodes to a respective signal inlet of a cardiac event recorder (18) :
- a ball forming element (42), disposed on the external side of the support band, comprising a bulged part (46) able to receive through clips a wire for connection to said recorder,
- a base forming element (44), disposed on the internal side of the support band, comprising a shank (52) that is crimped inside the ball forming element,
**characterized in that**
- the electrodes (34) are laterally prolonged by a flat tap able to establish an electrical connection and comprising a contact region (56) interposed between the internal side of the band (12) and the side opposite the base forming element (44) of the respective pushbutton, so as to establish an electrical connection between the pushbutton and the gel of the electrode, and
- the ball forming element (42) and the base forming element (44) of the pushbuttons enclose both the flat tab and the support band (12), through pinching the contact region (56) of the electrode (34) against the support band (12), and
- on the internal side a holding and protecting layer (62) covering the electrode's periphery and comprising a central window (55) revealing the active surface forming free part (68), facing the patient's body, of the electrode (34), is provided.

2. The thoracic belt according to Claim 1, wherein said holding and protecting layer (62) covers the free part, facing the patient's body, of the base forming element (44) of the pushbutton.

3. The thoracic belt according to Claim 1, wherein said holding and protecting layer is of a material having a gauze-like slack texture, that does not form any barrier between the gel of the electrode and the patient's body when the belt is placed in contact with the patient's thorax.

4. The thoracic belt according Claim 1, wherein the holding and protecting layer (62) is, at least in the region (64) extending on the electrode's periphery, coated with an adhesive material on the surface facing the electrode and the support band.

5. The thoracic belt according Claim 1, moreover comprising on the internal side an insulating removable film (40) that covers at least the free part (68), facing the patient's body, of the electrode.

6. The thoracic belt according to Claim 1, moreover comprising on the external side, in a forward position, a means (30) for fixing the housing of a cardiac activity recorder.

7. The thoracic belt according to Claim 1, comprising on the ends of the support band a means (26, 28) for closing and adjusting the length of the belt.

8. The thoracic belt according to Claim 1, wherein said solid gel is an adhesive gel.

9. The thoracic belt according to Claim 1, comprising on the internal side an insulating coating layer (60) covering the free part (58), facing the patient's body, of the base forming element of the pushbutton.

## Patentansprüche

1. Ein Brustgürtel (10) zur Erfassung von physiologischen Herzaktivitätssignalen, umfassend einen biegsamen Unterstützungsband (12), der geeignet ist, mit einer Schnalle auf dem Thorax eines Patienten geschlossen zu sein, was eine in Kontakt mit dem Körper des Patienten liegende Innenseite (22) und eine entgegengesetzte Aussenseite (24) definiert, wobei der Unterstützungsband :
- eine Vielzahl von in Form von festen Gelpastillen (34) gestalteten, auf vorbestimmten auf der Innenseite des Unterstützungsbandes liegenden Stellen angeordneten Erfassungselektroden (16), sowie
- eine entsprechende Vielzahl von in Form von metallischen Druckknöpfen gestalteten elektrischen Verbindungsgliedern (36), die jeweils für die Verbindung jeder der Elektroden an einen respektiven Signaleingang eines Herzereignisschreibers (18)
- ein kugelförmiges, auf der Aussenseite des Unterstützungsbandes angeordnetes Element (42), das ein ausgebauchtes Teil aufweist, das geeignet ist, durch Klipsen einen Verbindungsdraht zum besagten Schreiber aufzunehmen, und
- einen auf der Innenseite des Unterstützungsbandes angeordnetes, einen im inneren des kugelförmigen Elements eingefassten Schenkel (52) aufweisendes Basiselement (44), umfassen,
umfassen,
wobei der Brustgürtel **dadurch gekennzeichnet ist, dass**
- die Elektroden (34) seitlich durch eine flache Lasche verlängert sind, die geeignet ist, eine elektrische Verbindung herzustellen, und umfassend einen zwischen der Innenseite des Bandes (12) und der dem Basiselement (44) des respektiven Druckknopfs entgegengesetzten Seite zwischengelegten Kontaktbereich (56), um eine elektrische Verbindung zwischen dem Druckknopf und dem Gel der Elektrode herzustellen, und
- das kugelförmiges Element (42) und das Basiselement (44) der Druckknöpfe sowohl die besagte flache Lasche, als auch den Unterstützungsband (12) durch Einspannen des Kontaktbereichs (56) der Elektrode (34) an den Unterstützungsband (12) einschliessen, und
- eine den Umfang der Elektrode deckende und ein zentrales Fenster (66) aufweisende Aufrechterhaltungs- und Schutzschicht (62) auf der Innenseite vorgesehen sind, wobei das eine aktive Oberfläche (68) bildende, zum Körper des Patienten zugewandten, zur Elektrode (34) gehörenden freie Teil freigelegt bleibt.

2. Der Brustgürtel gemäss Anspruch 1, in welchem die besagte Aufrechterhaltungs- und Schutzschicht (62) ausserdem das freigelegte, zum Körper des Patienten zugewandten freie Teil des Basiselementes (44) des Druckknopfs deckt.

3. Der Brustgürtel gemäss Anspruch 1, in welchem die besagte Aufrechterhaltungs- und Schutzschicht aus einem Werkstoff gebildet ist, der eine lockere Struktur des Typs Gaze besitzt, wobei diese Schicht keinen Sperre zwischen dem Gel der Elektrode und dem Körper des Patienten bildet, wenn der Gürtel in Kontakt mit dem Thorax des Patienten angebracht wird.

4. Der Brustgürtel gemäss Anspruch 1, in welchem die Aufrechterhaltungs- und Schutzschicht (62) wenigsten im Bereich (64), der am Umfang der Elektrode ausdehnt, mit einem Klebstoff auf ihrer zur Elektrode und zur Unterstützungsband zugewandten Oberfläche beschichtet ist.

5. Der Brustgürtel gemäss Anspruch 1, umfassend ausserdem auf seiner Innenseite eine isolierende, ablösbare dünne Folie (40), die wenigstens das freie, zum Körper des Patienten zugewandte Teil (68) der Elektrode deckt.

6. Der Brustgürtel gemäss Anspruch 1, umfassend ausserdem auf seiner Aussenseite in vorderer Lage ein Mittel (30) zur Befestigung des Gehäuses eines Herzaktivitätsschreibers.

7. Der Brustgürtel gemäss Anspruch 1, umfassen auf den Enden des Unterstützungsbandes ein Mittel (26) zum Verschliessen und zum Einstellen der Länge des Gürtels.

8. Der Brustgürtel gemäss Anspruch 1, in welchem das besagte feste Gel ein Klebegel ist.

9. Der Brustgürtel gemäss Anspruch 1, umfassend auf seiner Innenseite eine Schicht eines das freie Teil (58) deckenden, zum Körper des Patienten zugewandten Isolierüberzugs (60).
